# EUROPEAN PATENT APPLICATION

(11) **EP 1 992 685 A1**
(43) Date of publication of application: **19.11.2008**
(21) Application number: 07740571.0
(22) Date of filing: 30.03.2007
(51) Int. Cl.: C12N 5/00, C12M 3/00, C12N 5/06

(54) **NOVEL CELL CULTURE METHOD AND METHODS OF PRODUCING AND COLLECTING CELL MASSES USING THE SAME**

(30) Priority: 31.03.2006 JP 2006100435
(71) Applicant: Asubio Pharma Co., Ltd., Minato-ku Tokyo 107-8541 (JP); Keio University, Tokyo 108-8345 (JP)
(72) Inventor: HATTORI, Fumiyuki, Mitaka-shi, Tokyo 181-0012 (JP); FUKUDA, Keiichi, Tokyo 160-8582 (JP)
(74) Representative: Vossius & Partner
(86) International application number: PCT/JP2007/057135
(87) International publication number: WO 2007/114351

(57) **Abstract**

The inventors of the present invention have developed a novel cell culture method with a view to providing a process by which cell masses uniform in size and properties can be produced in large quantities and with convenience, as well as a method of recovering them.

The inventors of the present invention found that the above-mentioned objects could be attained by using a structural member having a hollow portion at least one lower end of which is open, creating a projecting portion of a culture medium at the open lower end of the structural member, and culturing cells in that projecting portion of the culture medium. The present invention has been accomplished on the basis of this finding. Specifically, the present invention provides a cell culture method **characterized by** providing a structural member having a hollow portion at least one lower end of which is open, injecting a culture medium containing cells into the hollow portion of the structural member, causing a portion of the culture medium to project downward from the open end, and culturing the cells in the projecting portion of the culture medium. The present invention also provides a process for preparing cell masses **characterized by** forming cell masses by the above-described cell culture method.

## Description

### TECHNICAL FIELD

The present invention relates to a method for efficient production of a cell mass as an aggregate of highly uniform cells.

### BACKGROUND ART

Not a few kinds of cells, such as hepatocytes, may be subjected to two-dimensional culture to acquire properties that are different from those in vivo. For instance, when proteins produced from these cells or such cells per se are to be industrially utilized, it is known that they may be subjected to three-dimensional culture, which simulates culture conditions more similar to living tissues than two-dimensional culture, to form cell masses that can exhibit the functions of cells occurring in the living tissues (Non-Patent Document 1). To use the prepared cell masses and make them exhibit certain, say, hepatic functions for use in such applications as screening for therapeutics, it is necessary to prepare a cell group which is a copulation of homogenous cell masses that are uniform in external features such as size and shape, and in properties.

It is also known that as a convenient system for verifying the actions of anti-cancer drugs and the like, three-dimensional culture provides greater ease than two-dimensional culture in reflecting the properties of solid tumors, eventually allowing for greater accuracy in the assessment of drug efficacy (Non-Patent Document 2). To perform screening for anti-cancer drugs and the like using cell masses prepared by three-dimensional culture, it is necessary to obtain less noisy, positive results, which in turn requires homogeneity between cell masses to be used.

In addition, embryonic stem cells (also known as ES cells) do not differentiate in two-dimensional culture but if they are subjected to three-dimensional culture, a program for development and differentiation that involves the formation of cell masses known as embryoid bodies gets started and proliferating ES cells differentiate into multiple kinds of living tissue cells. When performing such differentiation into living tissue cells, homogeneous embryoid bodies may be prepared and used for appropriate differentiation into desired cells, which contributes to efficient differentiation into the desired cells.

As mentioned above, simply performing three-dimensional culture is insufficient from the viewpoint of obtaining homogeneous cell masses which are uniform in properties and external features such as size and shape, and industrially adequate results cannot be obtained from using the cell masses. Therefore, it is important to perform uniform three-dimensional culture and yield a group of homogeneous cell masses.

Various methods are conventionally known and used to form cell masses and they are divided into two major groups, one of which has been developed with a view to preparing embryoid bodies for differentiating embryonic stem cells and includes, for example, the hanging-drop method, the bacterial dish method, the swirl culture method, the bioreactor method, and the methyl cellulose method; the other group has been developed with a view to maintaining the properties of hepatocytes for a prolonged period and includes, for example, the sphere method and the encapsulation method.

To obtain a group of homogeneous cell masses, namely, to obtain a group of cell masses that are uniform in morphological features such as size and shape and in properties, several requirements must be satisfied, including the need to start every cell culture with the same cell count (which is hereinafter referred to as the initial cell count) and the need to ensure that the environment in the culture system for preparing cell masses (culture conditions such as the ingredients of a culture medium and the concentrations of dissolved gases) is kept constant during the culture period. However, the already known methods have such problems that although they can all form cell masses, no uniformity is obtainable among the cell masses prepared, and that there is no obtaining the efficiency or productivity that is required for industrial application.

In the bacterial dish method, cells are cultured on a plastic dish treated to prevent cell adhesion, whereby the suspended cells are allowed to make stochastic contact with themselves so that cell masses will gradually form. However, the number of cells that compose a single cell mass is not uniform in this method and there is eventually no forming a group of uniform cell masses.

The swirl culture method is a variation of the above-described bacterial dish method; when cells are cultured on a culture dish having low cell adherence, the dish is set in a swirling motion, whereby the suspended cells are allowed to make contact with themselves on more occasions than in the bacterial dish method so as to introduce uniformity in size among the cell masses formed. While this method is superior to the bacterial dish method in terms of the uniformity among cell masses, a problem still remains in terms of uniformity; in addition, the number of cell masses that can be prepared per unit area of culture is so small that the production efficiency is low and there is a problem from the viewpoint of industrial application.

The bioreactor method (Non-Patent Document 3) is similar to the swirl culture method in that the culture device is kept rotating in a constant direction but it differs from the swirl culture method in that the rotating direction is vertical. However, this method again has the same disadvantage as the swirl culture method; the number of cell masses that can be prepared per unit area of culture is so small and the production efficiency is so low that there is a problem from the viewpoint of industrial application.

In the methyl cellulose method, a thickener such as methyl cellulose is added to a cell suspension to suppress convection in the solution and the individual cells are cultured in a separated and suspended form, whereby single cells grow into cell masses. However, since the viscosity of the cell suspension is high enough to suppress convection in the solution, an environmental difference tends to occur between the part of the solution in contact with ambient air and the interior of the solution and this difference in culture conditions makes it difficult to form homogeneous cell masses.

In the sphere method, a cell suspension is injected into a cell culture plate having 96 round-bottom wells that has been surface treated to prevent cell adhesion and cells fall to the lowest part of the round bottom of each well under gravity, whereby cell masses are formed. In this method, a nearly constant volume of cell suspension can be injected into each well, so cell masses can be formed in each well on the basis of a uniform initial cell count, thus making it possible to prepare a group of comparatively uniform cell masses. However, problems remain in terms of efficiency, as exemplified by extreme difficulty involved in ensuring that the cell masses present on the bottom of each well are recovered without suffering any physical damage. In addition, increasing efficiency by applying a special treatment with a view to inhibiting cell adhesion to the plate surface results in a higher cost to cause a problem in terms of productivity.

The encapsulation method (Non-Patent Document 4 and Non-Patent Document 5) is **characterized in that** a plurality of cells are contained into gelled alginate capsules and allowed to proliferate within the gel to form cell masses. However, the use of chemicals for encapsulating alginate or agarose is essential and safety check must be made before the prepared cell masses are used for therapeutic purposes. In addition, the cell mass forming in each capsule after the plurality of cells have proliferated will combine with another cell mass to form an aggregate of cell masses; hence, the embryoid bodies taken out of the respective capsules have the problem of low uniformity.

In the hanging-drop method, a cell suspension is attached to the underside of a planar support and by the action of surface tension, drops of the cell suspension are held hanging from the underside of the planar support until cells fall under gravity to the lowest part of each of the hanging drops so that they agglomerate spontaneously, whereby cell masses are prepared (Non-Patent Document 6). Thus, by ensuring that a constant volume of cell suspension is attached to the underside of the planar support, the initial cell count for forming the individual cell masses can be made uniform with comparative ease and gas exchange with ambient air is effected on the surface of the solution where the cell masses are present; hence, among the above-described existing methods, the hanging-drop method provides the greatest ease by which the culture conditions for preparing cell masses are held uniform and this eventually makes it possible to form cell masses having the most uniform properties.

However, since this method allows the solution drops to hang from the underside of the planar support, the slightest vibration will cause the drops to fall or two adjacent drops to move horizontally to coalesce; hence, this method requires utmost care to operate. It is therefore essential that levelness be kept at all stages of the operation from attaching the solution to the underside of the planar support up until the recovery of cell masses; this involves the need to perform a careful manual operation and it is difficult to increase the efficiency and productivity by machine-based automation. What is more, in order to suppress the falling or coalescing of the hanging drops, an adequate space must be provided between adjacent drops but this puts limit on the number of cell masses that can be prepared per unit area of culture, making it difficult to perform high-volume production. If, on the other hand, a smaller volume of cell suspension is attached to the planar support in order to suppress the falling or coalescing of the hanging drops, it is difficult to secure the least minimum volume of culture medium that is required to culture cell masses in a favorable state for a certain period of time (for example, at least 20 µL which is a preferred volume of culture medium to be used for preparing embryoid bodies from ES cells). As described above, the hanging-drop method can prepare a group of homogeneous cell masses but involves a problem with the efficiency and productivity that are required for industrial application.
[Non-Patent Document 1]: Roberts RA and Soames AR, Fundam Appl Toxicol. 1993 21(2): 149-158
[Non-Patent Document 2]: Mellor HR et al., Br J Cancer. 2005 93(3): 302-309
[Non-Patent Document 3]: Gerecht-Nir S et al., Biotechnol Bioeng. 2004 86(5): 493-502
[Non-Patent Document 4]: Magyar JP et al., Ann N Y Acad Sci. 2001 944: 135-143
[Non-Patent Document 5]: Dang SM et al., Methods Mol Biol. 2005; 290: 353-364
[Non-Patent Document 6]: Singla DK and Sobel BE, Biochem Biophys Res Common. 2005 335(3): 637-42

### DISCLOSURE OF THE INVENTION

### PROBLEMS TO BE SOLVED BY THE INVENTION

The present invention has been accomplished in view of the above-described problems with the conventional cell culture methods and it aims to provide a cell culture method by which cells can be cultured uniformly and in large quantities, as well as a method by which a group of cell masses that are uniform in morphological features such as size and shape and in properties can be produced in large quantities and conveniently.

The inventors of the present invention found that the above-mentioned problems could be solved by using a structural member having a hollow portion at least one lower end of which is open, creating a projecting portion of a culture medium at the open lower end of the structural member, and culturing cells in that projecting portion of the culture medium. The present invention has been accomplished on the basis of this finding.

### MEANS FOR SOLVING THE PROBLEMS

The method of culturing cells according to the present invention is characterized by providing a structural member having a hollow portion at least one lower end of which is open, injecting a culture medium containing cells into the hollow portion of the structural member, causing a portion of the culture medium to project downward from the open end, and culturing the cells in the projecting portion of the culture medium.

In the present invention, the other end of the hollow portion may be closed such that the culture medium is injected into the hollow portion with the open end facing up, that a portion of the culture medium is caused to project upward from the open end, and that the open end is then caused to face down. Alternatively, in the present invention, the other end of the hollow portion may also be opened such that the culture medium is injected into the hollow portion, with the one open end facing down, through the other open end and that after the culture medium has been injected, the other open end is closed. Alternatively, in the present invention, the other end of the hollow portion may also be opened such that the culture medium is injected into the hollow portion, with the one open end being immersed in the culture medium, by sucking it from the other open end. Furthermore, in the present invention, a plurality of the hollow portions may be formed in the structural member so that a plurality of cell cultures can be performed simultaneously.

The process for producing cell masses according to the present invention is characterized by culturing cells by the above-described cell culture method to form cell masses. In this process for producing cell masses, the portion of the culture medium that projects downward from the one open end may be brought into contact with the solution's surface of another culture medium so that the cell masses are recovered into the another culture medium.

### BRIEF DESCRIPTION OF THE DRAWINGS

[FIG. 1]

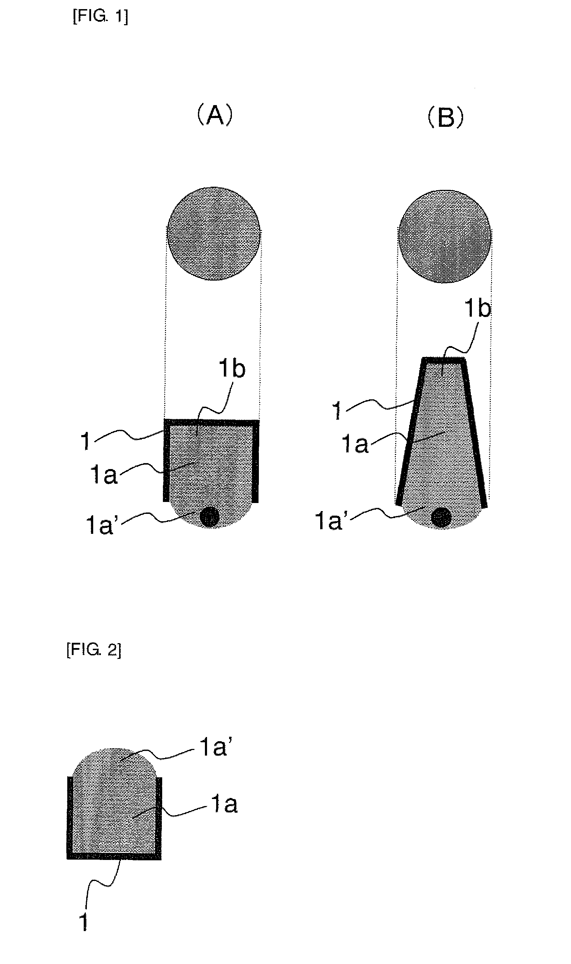

   FIG. 1 shows the new method provided by the present invention, in which a structural member (1) having a hollow portion (1b) that is open at the lower end is injected with a culture medium (1a), which is held projecting as indicated by (1a').
[FIG. 2]
   FIG. 2 illustrates a method which uses the structural member (1) shown in FIG. 1 to make the projecting portion (1a') of the culture medium.
[FIG. 3]

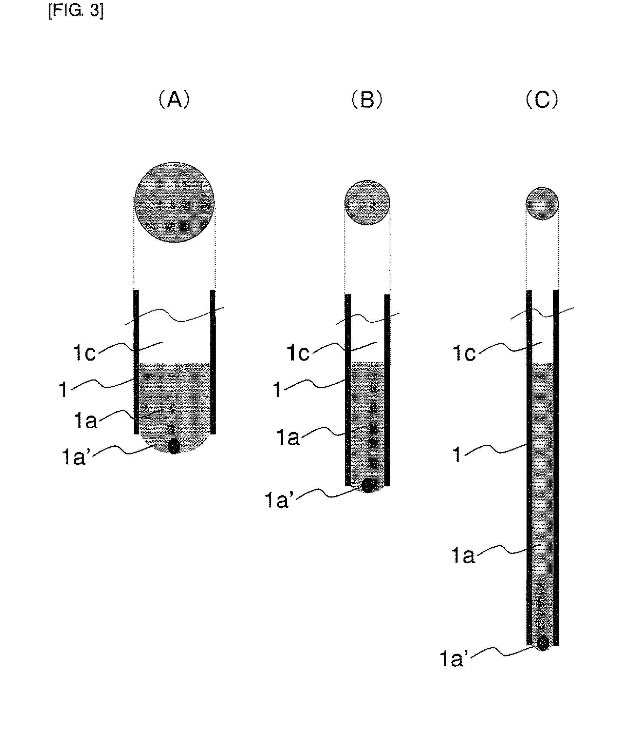

   FIG. 3 shows another embodiment of the new method provided by the present invention, in which a structural member (1) having a hollow portion (1c) that is open at both ends has a culture medium (1a) injected into the hollow portion (1c), which can be held projecting as indicated by (1a').
[FIG. 4]

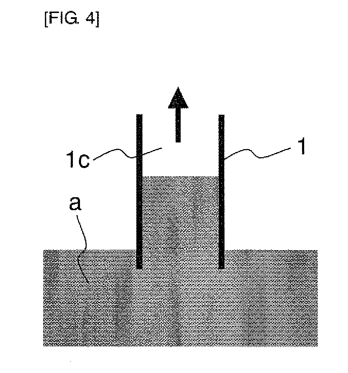

   FIG. 4 illustrates a method which uses the structural member (1) shown in FIG. 3 to make a projecting portion of a culture medium by sucking the culture medium (a) from the opening at the top.
[FIG. 5].

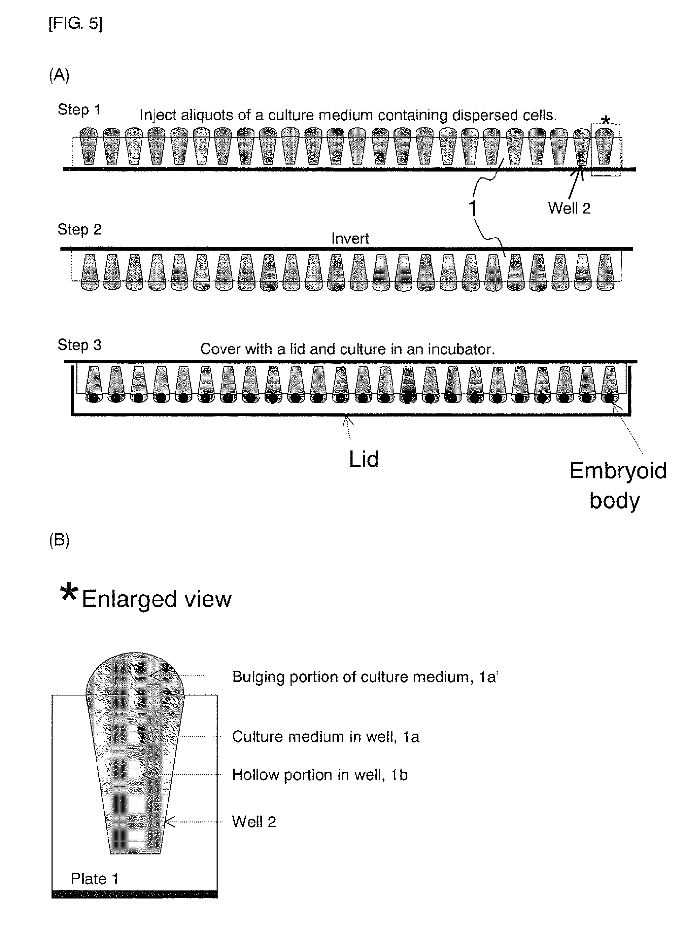

   FIG. 5 shows schematically (FIG. 5A) and partially enlarged (FIG. 5B) an application of a culture plate which is a convenient way to implement the culture method provided by the present invention.
[FIG. 6]

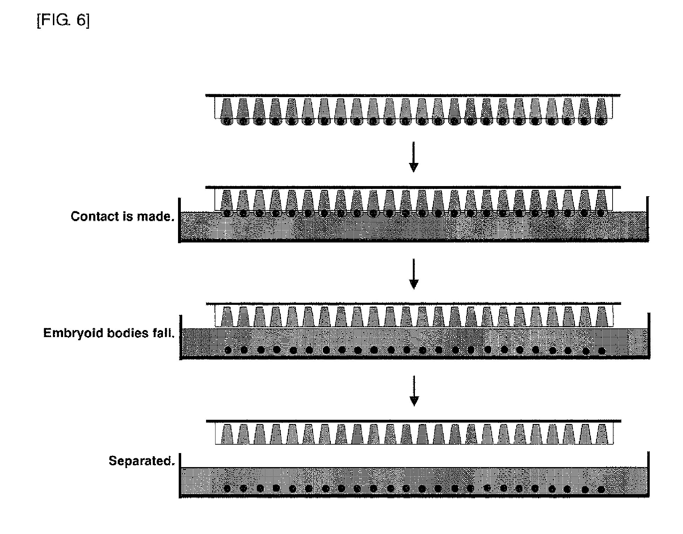

   FIG. 6 shows schematically the method described in Examples 1 and 2 which was used to recover the embryoid bodies as prepared cell masses into solution.
[FIG. 7]

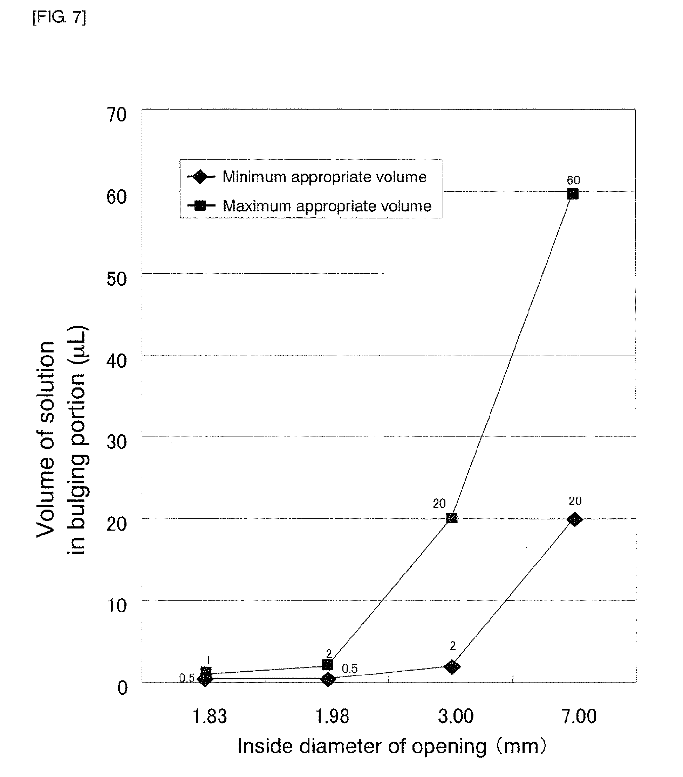

   FIG. 7 is a graph showing the relation for an existing plate between the inside diameter of the wells in the plate and the appropriate volume of a culture medium that can make a bulging portion of the culture medium to prepare cell masses.
[FIG. 8]

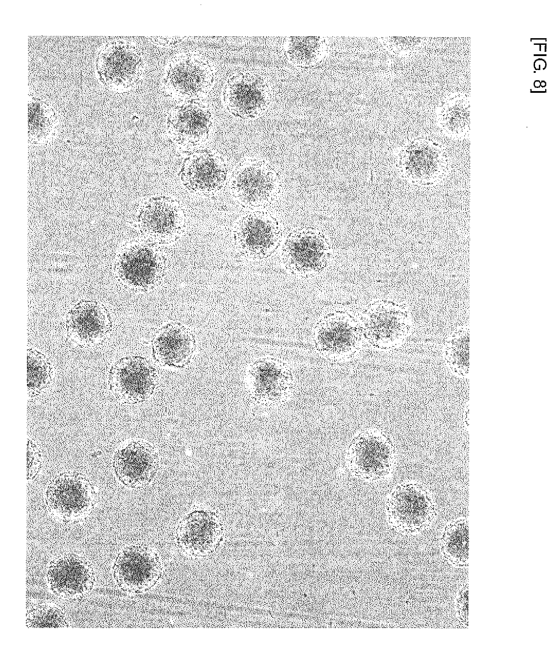

   FIG. 8 is a micrograph of the uniform embryoid bodies prepared in Example 2.

### LEGEND

1: a structural member having a hollow portion; 1a: a culture medium in the structural member having a hollow portion; 1a': a projecting portion of the culture medium; a: culture medium; 1b: a hollow portion that is open at one end and closed at the other end; 1c: a hollow portion that is open at both ends; 2: a well.

### MODES FOR CARRYING OUT THE INVENTION

On the following pages, the principle of the cell culture method of the present invention is described with reference to FIGS. 1 to 3.

In FIGS. 1A and 1B, a structural member (1) used in the cell culture method of the present invention is shown diagrammatically. Formed in the structural member (1) is a hollow portion (1b) into which a cell-containing culture medium (1a) is to be injected. The structural member (1) shown in FIG. 1A assumes the shape of a cup, turned upside down, that has a uniform diameter, with an end of the hollow portion (1b) (the lower end in FIG. 1) being open and the other end (the upper end in FIG. 1) being closed. The structural member (1) shown in FIG. 1B also assumes the shape of a cup turned upside down but it has a linearly changing diameter; again, the hollow portion (1b) is open at one end but closed at the other end. The closed end of the hollow portion (1b) may assume any shape; it may be angular as illustrated in FIGS. 1A and 1B or, alternatively, it may be rounded. The capacity of the hollow portion (1b) is determined in consideration of various factors including the kind of the cells to be cultured, the size of the cell masses to be finally produced.

The hollow portion (1b) is injected with the cell-containing culture medium (1a) in such a way that a portion of it indicated by (1a') projects downward from one end, namely, the open lower end, of the hollow portion. A method of injecting the culture medium into the hollow portion in this manner is shown in FIG. 2; the structural member (1) is first positioned in such a way that the open end of the hollow portion (1b) faces up; with that posture maintained, the culture medium (1a) is injected into the hollow portion (1b) in such a way that a portion of it indicated by 1a' projects, or bulges upward, from the open end of the hollow portion; thereafter, the structural member (1) is inverted, or turned upside down. In this way, the culture medium can be easily injected into the hollow portion of the structural member, and by appropriately determining the size (cross-sectional area) of the hollow portion in consideration of the viscosity and surface tension of the culture medium, the structural member can be inverted and yet the culture medium can be held in the state shown in FIG. 1.

By holding the culture medium (1a) within the hollow portion (1b) of the structural member (1) as shown in FIG. 1, the cells contained in the culture medium go down through it under gravity and collect in the portion (1a') projecting downward from the open end, eventually forming cell masses.

The structural member need not be of a cup shape as shown in FIG. 1; instead, it may be shaped like a tube or a pipe that is open at both ends as shown in FIG. 3. In FIGS. 3A to 3C, the hollow portion (1c) of the structural member (1) in a tubular or piped form is open at both an upper and a lower end. To inject the culture medium into this hollow portion with both ends open, the following method may be employed. To the upper end of the structural member (1), a culture medium supply source is connected via a supply pipe and a flow control valve, neither of which is shown, and the flow control valve is opened so that a predetermined volume of the culture medium is supplied from the culture medium supply source into the hollow portion (1c) of the structural member via the supply pipe; thereafter, the culture medium is so treated that its lower end projects downward from the open lower end of the hollow portion of the structural member, as indicated by 1a' in FIGS. 3A to 3C. This can be done by adjusting the volume of air being supplied from the upper open end of the hollow portion to the upper part of the culture medium.

Still another possible method is shown in FIG. 4; the lower end of the structural member is immersed in the culture medium (a) such that the lower open end of the hollow portion is plugged with the culture medium; a suction pipe (not shown) is connected to the upper end of the structural member (1) and air is drawn from the upper part of the hollow portion (1c) until a predetermined volume of the culture medium (a) is sucked into the hollow portion (1c) of the structural member (1); thereafter, the structural member (1) is lifted until its lower end comes off the liquid level of the culture medium.

According to the above-described method of the present invention, a predetermined volume of the culture medium is accommodated in the structural member, a portion of this culture medium is caused to project downward from the lower end of the structural member, or from the opening at the lower end of the hollow portion, and a cell mass is formed in that projecting portion of the culture medium; hence, compared to the conventional methods including the hanging-drop method which creates a bulge of solution on a planar substrate, the method of the present invention can realize efficient production of cell masses of uniform quality.

In order to produce cell masses efficiently by culturing cells in accordance with the method of the present invention, it is required to use a structural member having a hollow portion at least one lower end of which is open and creating a projecting portion of a cell suspension as a culture medium at the lower open end of the structural member. A cross section of the structural member (1) that is to accommodate a solution containing a group of cells is not limited to any particular shape as long as the solution having the cells suspended therein can be introduced into the structural member to create such a state that the solution bulges upward from the lower open end of the structural member.

The structural member may be made of any materials, such as polypropylene, polystyrene and silicone, that are used in the technical field of interest. Such materials are preferably selected from among those which permit only low cell adhesion, or those which have been surface treated for low cell adhesion.

In the next step, cells are cultured to form a cell mass in the projecting portion of the culture medium. To form a uniform cell mass, it is necessary that the number of cells contained in the culture medium (i.e., the total sum of 1a and 1a') which is to be accommodated within the hollow portion at the start of culture should be held constant. To ensure that the culture medium contains a constant number of cells, it is necessary that the cells which are to be cultured should be contained at a constant concentration within the culture medium and that the volume of the culture medium to be contained in the structural member having a hollow portion should be held constant. The concentration of the cells contained in the culture medium and the volume of the culture medium to be contained in the structural member having a hollow portion can appropriately be determined by skilled artisans considering various conditions including the growth rate of the cells, the size of the cell mass to be formed, and the number of days a culture is performed to form the desired cell mass.

The culture method of the present invention can be used with any kind of cells that are cultured to form a cell mass and the cells that can be applied are not limited in any particular way. For example, the culture method of the present invention is particularly suitable for culturing cells such as embryonic stem cells and hepatocytes that desirably form cell masses.

FIG. 5 shows a mode of embodiment in which a plurality of hollow portions are formed in a single planar structural member to thereby produce a multiple of cell masses at a time. In this mode of embodiment, a commercial cell culture plate having wells at high density (e.g., 384 wells or 1536 wells) is utilized as the structural member (1). The plate (1) as the structural member has multiple, say, 384 or 1536 hollow portions (1b) or wells 2 formed in an array. As shown in the top of FIG. 5A, the plate (1) is positioned in such a posture that the open end of each well faces up and a sufficient volume of a cell suspension as a culture medium to create a bulging surface is injected into each well (2) as indicated by 1a'; thereafter, the plate (1) is inverted upside down as shown in the middle of FIG. 5A, whereby a portion of the culture medium projects downward from the open end of each well; the cells in the culture medium (1a) collect in the bottommost part of the projecting portion (1a') of the culture medium to form a cell mass (as shown in the bottom of FIG. 5A). Testing of the above-described method showed that it was capable of preparing cell masses in large quantities at a very high density over a small space with rapidity and convenience. Thus, according to this mode of embodiment, productivity can be improved significantly over the conventional hanging-drop method, and since the area of contact between the culture solution and the structural member for culture is larger than in the hanging-drop method which utilizes stress like the method of the present invention, the solution in the process of culture can be sufficiently stabilized to enable homogeneous cell masses to be produced in large quantities and conveniently.

In the present invention, there is also provided a method in which the cell mass that has been formed by the above-described method in the projecting portion of the culture medium at the lower open end of the structure having a hollow portion is brought into contact with the solution surface of another culture medium, whereby the cell mass is recovered into the another culture medium (FIG. 6). By bringing the projecting portion of the culture medium into contact with the solution surface of another culture medium, the cell mass present in the projecting portion of the first culture medium can be spontaneously moved to precipitate in the another culture medium, whereby the cell mass can be recovered from the plate with great ease.

Thus, it has been verified that the cell masses prepared by the method of the present invention are not only extremely uniform in size (FIG. 8) but that homogeneous caridomyocytes can also be prepared from those cell masses prepared with ES cells (i.e., embryoid bodies); it has also been confirmed that the thus prepared cell masses are homogeneous. As a further advantage of the method of the present invention, a large number of projecting portions of culture medium can be prepared per unit area (this has been impossible in the prior art methods) and yet the operation of making the projecting portions of culture medium can be done rapidly and conveniently to show a marked improvement in the efficiency and productivity of the preparation of cell masses.

To be more specific, it is required in the hanging-drop method that a smaller volume of cell suspension should be attached to the planar support in order to prepare more cell masses per unit area; on the other hand, it has been necessary to secure the required volume of culture medium for allowing the cell masses to be cultured in a satisfactory state for a certain period of time and this has put a limit on the number of cell masses that can be prepared per unit area. In contrast, in the method of the present invention, the volume of solution in the projecting portion of the culture medium (the area indicated by 1a' in FIG. 5B) can be set independently of the volume of solution in the structural member having a hollow portion (the area indicated by 1a in FIG. 5B); hence, even if the size of the projecting portion of culture medium is reduced, one may increase the volume of solution in the structural member having a hollow portion to secure the required volume of culture medium in the culture for preparing cell masses.

In the hanging-drop method, it has also been necessary to attach the cell suspension to the surface on the underside of the planar substrate, and this has rendered the operation of attaching the solution to be a complicated task that requires much time and labor. In contrast, in the method of the present invention, the step of injecting the cell culture medium as a culture suspension into each of structural bodies having a hollow portion can be automated with a machine and the operation of making the projecting portions of culture medium can be greatly facilitated.

The method of the present invention has additional advantages: since the cell masses being cultured simultaneously can undergo gas exchange with the ambient air on the surface of the projecting portion of culture medium, so it is easy to hold the culture conditions constant; and what is more, the number of cells to be used to form each cell mass can also be easily held constant. In the method of the present invention, the surface tension developing between the lower open end and the projecting portion of the culture medium is markedly stronger than the surface tension occurring between the plane used in the hanging-drop method and the cell suspension attached to this plane, so there is offered still another advantage that the projecting portion of the culture medium at the open end remains stable even if the plate into which the culture medium has been injected is inverted upside down.

The present inventors also studied the relation between the inside diameter of the structural body having a hollow portion used as a culture vessel in the present invention and the volume of a culture medium in its projecting portion (corresponding to the area indicated by 1a' in FIG. 5B), the results being shown in FIG. 7. When the inside diameter of the opening was 1.83 mm, 1.98 mm, 3.00 mm and 7.00 mm, the appropriate volume of the culture medium in the projecting portion was 0.3-1.5 µL, 0.5-2.0 µL, 2.0-20 µL, and 20-60 µL, respectively. Based on the graph shown in FIG. 7, any skilled artisan can easily determine the appropriate volume of the culture medium in its projecting proportion. For example, at least 20 µL of the culture medium is preferably required to culture 100 mouse embryonic stem cells for 2 days. In other words, if the hollow portion of a structural member that is to be used in the present invention has such an inside diameter at the opening that the appropriate volume of a culture medium in its bulging portion is less than 20 µL, the deficiency can be compensated by the culture medium to be accommodated in that structural member. It is therefore desirable to use in the present invention a structural member (culture device) having such a hollow portion that it provides an internal space of at least 20 µL in structure.

### EXAMPLES

### Example 1: Reparation of Mouse ES Cell Derived Embryoid Bodies Using a 384-Well Plate

In this example, embryoid bodies were prepared from mouse ES cells by the method of the present invention using a commercial 384-well cell culture plate, with each well serving as a structural member having a hollow portion; the purpose of this example was to investigate the number of cell masses that could be prepared per unit area of the plate and the efficiency of preparing a group of cell masses.

In this example, a cell line called EB3 (Niwa H et al., Nat Genet 2000; 24: 372-376) was used as ES cells, which were diluted with an α-MEM culture medium (Sigma) supplemented with immobilized (55°C, 30 min) fetal bovine serum having a final concentration of 10% to give a concentration of 75 ES cells/35 µL.

Subsequently, the aliquots of the thus prepared mouse ES cells were injected on a commercial 384-well cell culture plate (product of Greiner, Model 788161; opening's inside diameter at 3.0 mm) and embryoid cells were prepared in accordance with the following method. The nominal volume of solution that was permissible for the 384-well plate used was 25 µL per well but in order to make the solution's surface bulge upward, 35 µL of the ES cell containing culture medium was injected into each well. In this way, 75 ES cells were injected into each well. On this occasion, the volume of the solution that was required to fill up to the level surface at the opening was 28 µL and the volume of the solution that was required to make the bulge was 7 µL. For the divided injection, a multi-channel pipette of Theremo Labsystems (Product No. 4610070) or a dispensing machine of Biotech Lab. Inc. (Model LD-01) was used.

The plate into which the aliquots of the culture medium containing ES cells had been injected to make the bulges of the culture medium was turned upside down to make a projecting portion of the culture medium at each opening on the bottom. With this state being kept intact, the plate was closed with a lid and culture was performed in an incubator under conditions of 37°C and 5% CO₂ (FIG. 5) to have ES cells grow in the projecting portions at the bottom openings of the plate. One day after the start of culture, the plate with the projecting portions of the culture medium facing down was held with a clean device such as tweezers and put into a larger vessel filled with an α-MEM culture medium (Sigma) supplemented with immobilized (55°C, 30 min) fetal bovine serum having a final concentration of 10%, so that the projecting portions of the culture medium were brought into contact with the surface of the α-MEM culture medium, whereupon the cell masses precipitated under gravity into the culture medium in the larger vessel, thus enabling recovery of the ES cell derived cell masses, or embryoid bodies (FIG. 6). The embryoid bodies obtained were substantially uniform in size as shown in FIG. 8.

When the 384-well plate and the dispensing machine of Biotech Lab. Inc. were employed, the aliquots of the ES cell containing culture medium could be injected on the plate at a rate of 24 cells per second in Example 1, although this value would vary with settings on the machine such as those of divided injection. From this result, it became clear that the time required to make the projecting portions of the culture medium could be considerably reduced compared to the case of the conventional hanging-drop method. It was also possible to prepare large amounts of embryoid bodies of substantially uniform size. Thus, the present invention could provide a method by which cell masses can be produced and recovered in large quantities with rapidity in an industrially applicable high efficiency.

### Example 2: Preparation of Mouse ES Cell Derived Embryoid Bodies Using a 1536-Well Plate

In this example, embryoid bodies were prepared from mouse ES cells by the method of the present invention using a commercial 1536-well cell culture plate, with each well serving as a structural member having a hollow portion; the purpose of this example was to investigate the number of cell masses that could be prepared per unit area of the plate and the efficiency of preparing a group of cell masses.

As in Example 1, EB3 cell line was used as ES cells in Example 2 and they were diluted with an α-MEM culture medium (Sigma) supplemented with immobilized (55°C, 30 min) fetal bovine serum having a final concentration of 10% to give a concentration of 75 ES cells/12.4 µL.

Subsequently, the aliquots of the thus prepared mouse ES cells were injected on a commercial 1536-well cell culture plate (product of Genentix, Model X1536 PS; opening's inside diameter at 1.83 mm) and embryoid cells were prepared in accordance with the following method. The nominal volume of solution that was permissible for the 1536-well plate used was 8 µL per well but in order to make the solution's surface bulge upward, 12.4 µL of the ES cell containing culture medium was injected into each well. On this occasion, the volume of the solution that was required to fill up to the level surface at the opening was 11 µL and the volume of the solution that was required to make the bulge was 1.4 µL. Thus, 75 ES cells were injected into each well. For the divided injection, a dispensing machine of Biotech Lab. Inc. (Model LD-01) was used.

As in Example 1, the aliquots of the culture medium containing ES cells was injected, ES cells were cultured in the projecting portions at the bottom openings of the plate, and the ES cell derived cell masses, or embryoid bodies were recovered. As in Example 1, the embryoid bodies obtained were substantially uniform in size.

When the 1536-well plate and the dispensing machine of Biotech Lab. Inc. were employed, the aliquots of the ES cell containing culture medium could be injected on the plate at a rate of 28 cells per second in Example 2, although this value would vary with settings on the machine such as those of divided injection. From this result, it became clear that in Example 2, the time required to make the projecting portions of the culture medium could also be considerably reduced compared to the case of the conventional hanging-drop method. It was also possible to prepare large amounts of embryoid bodies of substantially uniform size. Thus, the present invention could provide a method by which cell masses can be produced and recovered in large quantities with rapidity in an industrially applicable high efficiency.

### Example 3: Study on the Inside Diameters of Various Plates and the Appropriate Solution's Volume Required to Form the Projecting Portion of Culture medium

In this example, various commercial cell culture plates were used to prepare cell masses by forming projecting portions of a culture medium and then inverting the plates; the purpose of this example was to study the relation between the inside diameter of the openings in each plate and the appropriate solution's volume that was required to form the projecting portions but which would not allow the culture medium to drop even when the plate was inverted.

As in Example 1, an α-MEM culture medium (Sigma) supplemented with immobilized (55°C, 30 min) fetal bovine serum having a final concentration of 10% was used in Example 3.

In this example, four commercial cell culture plates were used: Genentix 1536-well plate (X1536 PS; inside diameter at 1.83 mm); Nunc 384-well plate (164320); Grenier 384-well plate (788161; inside diameter at 3.00 mm); and SUMITOMO BAKELITE 96-well plate (MS -0096S; inside diameter at 7.00 mm). When the Nunc 384-well plate (164320) was used, the smaller wells (inside diameter at 1.98 mm) between larger wells (inside diameter at 3.00 mm) were utilized. To determine the inside diameter of the well openings in each plate, a microphotograph was taken of wells in each plate and using the simultaneously taken scale for microscope, the diameter of the well openings in each plate was measured.

In the next place, varying volumes of the culture medium were injected into the wells in each plate and the appropriate solution's volume that was required to form the projecting portion of the culture medium was investigated; the '"appropriate" means that the solution's surface of the culture medium bulged upward and that even when the plate on which the aliquots of the culture medium was injected was inverted, the culture both would not spill but formed a projecting portion at the lower open end and enabled cell culture to be done.

The results are shown in FIG. 7. While various volumes of the solution were required to make a bulge after the well was completely filled of the solution (when the top surface of the well was level with the solution's surface), FIG. 7 shows guide figures for the ranges of the appropriate solution's volume over which the stability of the projecting portion was evaluated. As it turned out, when the following conditions were met, the projecting portion of the culture medium could be held stable even when it was inverted to face down: 0.3-1.5 µL, preferably 1 µL, for the 1536-well plate (i.d. 1.83 mm); 0.5-2 µL, preferably 1.5 µL, for the 384-well plate (i.d. 1.98 mm); 2-20 µL, preferably 15 µL, for the 384-well plate (i.e. 3.0 mm); and 10-60 µL, preferably 50 µL, for the 96-well plate (i.d. 7.00 mm).

### INDUSTRIAL APPLICABILITY

It has become clear that by using the method of the present invention, the time required to create a portion of a culture medium that projects from the structural member (i.e., the culture support) can be considerably reduced as compared with the case of the conventional hanging-drop method. In addition, homogeneous embryoid bodies which are substantially uniform in external features such as size and in properties could be prepared easily and in large quantities. In other words, according to the present invention, cell masses could be produced and recovered in large quantities and with rapidity in an industrially applicable high efficiency.

## Claims

1. A cell culture method **characterized by**
providing a structural member having a hollow portion at least one lower end of which is open;
injecting a culture medium containing cells into the hollow portion of the structural member;
causing a portion of the culture medium to project downward from the open end; and
culturing the cells in the projecting portion of the culture medium.

2. The cell culture method according to claim 1, **characterized in that** the other end of the hollow portion is closed, that the culture medium is injected into the hollow portion with the open end facing up, that a portion of the culture medium is caused to project upward from the open end, and that the open end is then caused to face down.

3. The cell culture method according to claim 1, **characterized in that** the other end of the hollow portion is also opened, that the culture medium is injected into the hollow portion, with the one open end facing down, through the other open end, and that after the culture medium has been injected, the other open end is closed.

4. The cell culture method according to claim 1, **characterized in that** the other end of the hollow portion is also opened, and that the culture medium is injected into the hollow portion, with the one open end being immersed in the culture medium, by sucking it from the other open end.

5. The cell culture method according to any one of claims 1 to 4, **characterized in that** a plurality of the hollow portions are formed in the structural member so that a plurality of cell cultures can be performed simultaneously.

6. A process for producing cell masses, **characterized in that** cells are cultured by the cell culture method according to any one of claims 1 to 5 to form cell masses.

7. A process for producing cell masses, **characterized in that** embryonic stem cells are cultured by the cell culture method according to any one of claims 1 to 5 to form embryoid bodies.

8. The process for producing cell masses according to claim 6 or 7, **characterized in that** the portion of the culture medium that projects downward from the one open end is brought into contact with the liquid surface of another culture medium so that the cell masses are recovered into the another culture medium.

9. A culture device for use in the cell culture method according to any one of claims 1 to 5 or the process for producing cell masses according to any one of claims 6 to 8.
